# EUROPÄISCHE PATENTSCHRIFT

(11) **EP 1 562 965 B1**
(45) Veröffentlichungstag und Bekanntmachung des Hinweises auf die Patenterteilung: **05.04.2006**
(21) Anmeldenummer: 03789016.7
(22) Anmeldetag: 07.11.2003
(51) Int. Cl.: C07H 7/027

(54) **VERFAHREN ZUR HERSTELLUNG VON 2-KETO-L-GULONSÄURE-C-4 C-10 /sb -ALKYLESTERN**
METHOD FOR THE PRODUCTION OF 2-KETO-L-GULONIC ACID-C-4-C-10 /sb ALKYL ESTERS
PROCEDE DE PRODUCTION D'ESTERS D' ALKYLE C-4 -C-10 /sb D ACIDE 2-CETO-L-GULONIQUE

(30) Priorität: 11.11.2002 DE 10252659
(43) Veröffentlichungstag der Anmeldung: 17.08.2005
(73) Patentinhaber: BASF Aktiengesellschaft, 67056 Ludwigshafen (DE)
(72) Erfinder: DOMSCHKE, Thomas, 67346 Speyer (DE); MERGER, Martin, 67061 Ludwigshafen (DE); GROSSMANN, Georg, 68199 Mannheim (DE); FAUST, Tillmann, 67256 Weisenheim (DE)
(86) Internationale Anmeldenummer: PCT/EP2003/012458
(87) Internationale Veröffentlichungsnummer: WO 2004/043880

(56) Entgegenhaltungen:
- EP-A- 0 403 351
- WO-A-01/85711
- WO-A-97/43433
- DE-A- 19 954 511

## Beschreibung

2-Keto-L-gulonsäureester stellen wichtige Zwischenprodukte für die Synthese von L-Ascorbinsäure (Vitamin C) dar. Die Veresterung von 2-Keto-L-gulonsäure (KGS) mit einem niederen Alkohol ist aus zahlreichen Publikationen bekannt. In der Regel wird zunächst ein C₁-C₄-Ester der KGS hergestellt, da dieser in organischen Lösungsmitteln besser löslich ist als KGS und mit hoher Selektivität in Ascorbinsäure oder Ascorbat überführt werden kann, bevorzugt werden Methyl- und Butylester verwendet.

Grundsätzlich wird bei diesem Reaktionstyp die Ausbeute durch das Veresterungsgleichgewicht limitiert. Die Vollständigkeit der jeweiligen Veresterung (Veresterungsgrad) wird in erster Linie durch den Restwassergehalt im Reaktionsgemisch bestimmt, da die Veresterungsreaktion im Gleichgewicht mit der Rückreaktion (Hydrolyse) steht.

Bei den üblichen Verfahren, u.a. dem Reichstein Verfahren, wird zur Erzielung eines hohen Veresterungsgrades und damit einer befriedigenden Ausbeute üblicherweise eine lange Kochzeit gewählt. Diese wirkt sich jedoch zu Lasten der Reinheit aus, da sowohl die 2-Keto-L-gulonsäure als auch ihr Methylester unter diesen Bedingungen weitere Nebenprodukte bilden können.

In vielen bekannten Verfahren profitiert die Veresterung von einem Alkohol-Überschuß oder von einem kontinuierlichen Entfernen des gebildeten Wassers.

Um den Wassergehalt bei der Veresterung zu reduzieren, sind verschiedene Methoden vorgeschlagen worden. Wasser kann während der Reaktion durch fortwährendes Abdestillieren des Wasser/Alkoholgemisähes, Behandeln des Destillates mit Molekularsieben und Rezyklisierung des so getrockneten Alkohols entfernt werden (Pol. Pat. 57042; Pol. Pat. 57573). Als eine andere Möglichkeit wurde das während der Reaktion fortwährende Abdestillieren des Wasser/Alkoholgemisches und Ersatz mit frischem trockenem Alkohol erwähnt (EP-A-0 535 927). Dabei muß in beiden Fällen eine große Menge Alkohol mit entsprechend großem Energieaufwand abdestilliert werden. Außerdem werden Reaktionszeiten von bis zu 10 Stunden notwendig, mit der Gefahr von Zersetzung und Nebenreaktionen.

EP-A-0 671 405 beschreibt ein Verfahren zur Herstellung von 2-Keto-L-gulonsäure-methyl- oder -ethylester durch Veresterung von 2-Keto-L-gulonsäure mit Methanol bzw. Ethanol in Gegenwart eines sauren Ionenaustauschers.

EP-A-0 403 993 beschreibt ein Verfahren zur Herstellung von 2-Keto-L-gulonsäure-methylester, bei der die Veresterung nur partiell, d.h. nicht bis zur Erreichung des Veresterungsgleichgewichts durchgeführt wird.

In einem in WO 99/03853 beschriebenen Verfahren wird 2-Keto-L-gulonsäure in einem zweistufigen Veresterungsprozeß zu 2-Keto-L-gulonsäureester umgesetzt, wobei die nach dem ersten Veresterungsschritt entstehende Lösung zur Entfernung des gebildeten Reaktionswassers wenigstens teilweise eingedampft und der entstandene Rückstand einem zweiten Veresterungsprozeß unterzogen wird.

WO 97/43433 sowie US 5,391,770 beschreiben die Synthese von 2-Keto-L-gulonsäure-butylester durch mehrstündiges Rückflußkochen von 2-Keto-L-gulonsäure in Butanol in Gegenwart von p-Toluolsulfonsäure und anschließendes Auskristallisieren des Wertproduktes durch Abkühlen der Reaktionsmischung.

DE-A-198 29 809 betrifft ein Verfahren zur Herstellung von Estern aus Alkohol und Carbonsäure mittels eines Katalysators und Abtrennung des Esters in einer mit Einbauten versehenen Rektifikationskolonne.

DE 199 38 980 beschreibt ein Verfahren bei dem ein C₁- bis C₁₀-Alkylester der KGS dadurch gewonnen wird, dass die Veresterungsreaktion in einem Flüssigkeitsfilm an einer heissen Oberfläche bei gleichzeitiger Abtrennung von Wasser durchgeführt wird.

Da die Siedetemperatur von Methanol weit unter der von Wasser liegt, kann Wasser aus einem Wasser/Methanol-Gemisch der Reaktionsmischung nur unter sehr großem energetischen Aufwand in einer Reaktivdestillation entfernt werden (Abdestillation eines Me-OH/Wasser-Gemisches mit nur geringem Wasseranteil, Kondensation, Entwässerung und Rückführung großer Methanolmengen). Dies führt dazu, dass in technisch ausgeübten Verfahren die KGS vor der Veresterung in aufwendiger Weise kristallisiert oder gefällt, getrocknet und calciniert wird. Bei der anschließenden Veresterung verbleibt das Reaktionswasser im Gemisch, der Veresterungsgrad bleibt unter 100 %, wodurch die Gesamtausbeute dieses Verfahrens vermindert wird. Die C₂- und C₃-Alkohole zeigen ähnliches Stoffverhalten und kommen technisch daher kaum zum Einsatz.

Höhere Alkohole, d.h. C₄- bis C₁₀-Alkylalkohole, speziell die C₄-Alkohole, und hier insbesondere 1-Butanol, bilden mit Wasser meist ein Azeotrop. Beim n-Butanol existiert ein Hetero-Azeotrop, das erhebliche Mengen Wasser enthält (ca. 40 % (m/m)). Hier kann daher während der Veresterung, unter vergleichsweise geringem Energieaufwand, in situ Wasser aus der Reaktionsmischung durch Destillation unter Rückführung der BuOH-Phase des kondensierten Heteroazeotropes, entfernt werden. Dies hat zwei wesentliche Vorteile:
1. Die KGS muß weder calciniert noch getrocknet werden, sie kann sogar als wässrige Lösung in den Prozess eingebracht werden.
2. Die Veresterung kann nahezu vollständig vollzogen werden, da auch das Reaktionswasser aus der regierenden Mischung entfernt weren kann.

KGS besitzt in den genannten höheren C4-C10-Alkoholen eine geringere Löslichkeit als in Wasser. Eine Entfernung des Wassers während der Veresterungsreaktion gemäß den Verfahren, die Stand der Technik beschrieben werden, führt daher i.d.R. zu KGS-Ablagerungen, da mit der Reduzierung des Wasseranteils in der Veresterungsreaktion KGS ausfällt. Um die Feststoffablagerungen zu entfernen, müssen Verfahren regelmäßig runter gefahren und Apparate gereinigt werden. Es kommt zu Ausfallzeiten und Produktverlusten.

Der vorliegenden Erfindung liegt nun das Problem zugrunde, dass es bei den im Stand der Technik beschriebenen Verfahren zur Herstellung von Ascorbinsäure unter Verwendung von C4- oder höheren Alkylestern als Zwischenprodukten, regelmäßig zu Feststoffablagerungen kommen kann. Das hat zur Folge, dass die Ausbeute, Standzeit und Betriebssicherheit des Verfahrens erheblich beeinträchtigt werden.

Die Aufgabe der vorliegenden Erfindung ist es somit, ein vorteilhaftes Verfahren zur Verfügung zu stellen, um 2-Keto-L-gulonsäure-C₄-C₁₀-alkylester herzustellen, ohne die aus dem Stand der Technik bekannten Nachteile, wie z.B. hoher Alkylesterverbrauch, geringe Ausbeute, lange Reaktionszeiten, Verfärbungen sowie erheblichem Nebenproduktanteil und der insbesondere geeignet zur Umsetzung zu L-Ascorbinsäure ist.

Das der Erfindung zugrunde liegende Problem wird folgend durch die hierin beschriebenen und in den Ansprüchen charakterisierten Ausführungsformen gelöst.

Folglich betrifft die Erfindung ein Verfahren zur Herstellung von 2-Keto-L-gulonsäure-C₄-C₁₀-alkylester durch Veresterung von 2-Keto-L-gulonsäure (KGS) mit einem C₄-C₁₀-Alkohol, das dadurch gekennzeichnet ist, dass in einer Vorveresterung eine wässrige KGS-Lösung mit einem gesättigten, verzweigten oder unverzweigten C₄-C₁₀-Alkohol unter saurer Katalyse bis zu einem Veresterungsgrad von 20 % bis 70 %, bevorzugt von 30% bis 50 %, umgesetzt wird; und das Produkt in einem kontinuierlichen Rektifikationsapparat mit einem C₄-C₁₀-Alkohol entwässert wird, wodurch die Veresterungsreaktion fortschreitet. Der Rektifikationsapparat kann eine ein- oder mehrstufige Destillationsapparatur sein, wie sie umfangreich im Stand der Technik beschrieben ist. Insbesondere ist der Rektifikationsapparat so ausgelegt, dass in dem Apparat die Veresterung erfindungsgemäß durchgeführt werden kann. Vorteilhaft ist folglich ein Rektifikationsapparat mit hohen Verweilzeiten der Lösung auf den Böden, wie unten beschrieben.

Zur Veresterung eignen sich prinzipiell alle C₄-C₁₀-Alkohole, vorteilhafterweise gesättigte, verzweigte oder unverzweigte Alkylalkohole mit einer Kohlenstoffanzahl größer gleich 4 bis 10 Kohlenstoffatomen, beispielsweise 1-Butanol, 2-Butanol, 2-Methyl-1-propanol, 2-Methyl-2-propanol, 1-Pentanol, 2-Pentanol, 3-Pentanol, 1-Hexanol, 2-Hexanol, 1-Heptanol, 2-Heptanol, 1-Octanol, 2-Octanol, 3-Octanol, 1-Nonanol, 2-Nonanol, 1-Decanol, 2-Decanol, 4-Decanol, besonders bevorzugt C₄-C₈-Alkohole, ausgewählt aus der Gruppe, bestehend aus 1-Butanol, 2-Butanol, 2-Methyl-1-propanol, 2-Methyl-2-propanol, 1-Pentanol, 1-Hexanol und 1-Octanol. In einer bevorzugten Ausführungsform ist der zur Veresterung und Entwässerung verwendete Alkohol ein C₄- bis C₆-Alkohol. Ganz besonders bevorzugter Alkohol ist ein C₄-Alkohol, insbesondere n-Butanol.

In der Vorveresterung wird in einer bevorzugten Ausführungsform der Alkohol dabei in einem Massenverhältnis zur wässrigen KGS-Lösung von 1:1 bis 5:1, bevorzugt von 2,5:1 bis 3,5:1 eingesetzt.

In einer weiteren Ausführungsform wird das Verfahren dadurch charakterisiert, dass zur sauren Katalyse ein homogener oder heterogener saurer Katalysator verwendet wird. Durch die Verwendung eines Katalysators kann die Reaktionstemperatur gering gehalten werden und somit die Entstehung von thermischen Zersetzungsprodukten vermieden werden.

Durch Zusatz eines sauren Katalysators wird die Veresterungsreaktion in an sich bekannter Weise katalysiert.

Als Veresterungskatalysatoren können in der Regel alle an sich bekannten homogenen oder heterogenen sauren Katalysatoren eingesetzt werden.

Als homogene Katalysatoren sind beispielsweise Mineralsäuren bzw. deren Ester geeignet. Dazu zählen insbesondere Phosphorsäure, Phosphorsäure-monobutylester, Phosphorsäure-dibutylester, Phosphorsäure-monopentylester, Phosphorsäure-dipentylester, Schwefelsäure, Schwefelsäure-monobutylester, Schwefelsäure-monopentylester, Chlorwasserstoff, p-Toluolsulfonsäure, Methansulfonsäure, Trifluormethansulfonsäure, Chlorsulfonsäure und Trifluoressigsäure. Auch 2-Keto-L-gulonsäure oder Ascorbinsäure können als Veresterungskatalysatoren eingesetzt werden.

Bevorzugt verwendet man Schwefelsäure, p-Toluolsulfonsäure, Methansulfonsäure oder Monoalkylsulfate der verwendeten Alkohole. Die Monoalkylsulfate spalten bei Temperaturen oberhalb von 70°C Schwefelsäure ab, die dann katalytisch wirkt. Besonders bevorzugte homogene saure Katalysatoren sind Schwefelsäure und p-Toluolsulfonsäure.

Im Falle der o.g. homogenen Katalysatoren können diese entweder einem der Edukte vor der Reaktion zugemischt werden oder als separater Strom in den Reaktor eingespeißt werden.

Heterogene Katalysatoren sind vorteilhafterweise im Reaktor in der heißen Reaktionszone fixiert. Für den Einbau heterogener Katalysatoren in Destillationskolonnen sind in der Literatur zahlreiche Konstruktionsmöglichkeiten beschrieben. Hierzu gehören Verweilzeitböden, bei denen der Katalysator auf den Böden oder in deren Ablaufschächten angeordnet sein kann, ferner beschichtete Füllkörper, gewickelte und strukturierte Packungen mit eingewebtem Katalysator.

Als heterogene Katalysatoren kommen die an sich bekannten sauren Katalysatoren, bevorzugt saure Ionentauscher als auch Zeolithe, in Frage.

Unter der Bezeichnung "saure Kationenaustauscher" sind kommerziell erhältliche Harze bzw. Detoxane zu verstehen, wie z.B. Lewatit® S 100, SP 112 oder Lewatit® 2631 (Bayer) oder Amberlite® 18 und IRA 120 oder Amberlyst® 15 oder Duolite® C 20, C 26 und C 264 (Rohm & Haas) oder Dowex® Ionentauscher.

Zeolithe sind kristalline Aluminosilikate, die eine hochgeordnete Struktur mit einem starren dreidimensionalen Netzwerk von SiO₄₋oder AlO₄-Tetraedern besitzen, die durch gemeinsame Sauerstoffatome verbunden sind. Das Verhältnis der Si- und Al-Atome zu Sauerstoff beträgt 1:2. Die Elektrovalenz der Aluminium enthaltenden Tetraeder ist durch Einschluß von Kationen in den Kristall, z.B. eines Alkali- oder Wasserstoffatoms ausgeglichen. Ein Kationenaustausch ist daher möglich. Die Räume zwischen den Tetraedern sind vor der Dehydration durch Trocknen bzw. Calcinieren von Wassermolekülen besetzt.

Geeignete Zeolithe sind z.B. solche vom Pentasil-Typ, besonders Aluminosilikat-Zeolithe oder Borosilikat-Zeolithe. Durch partielle Verkokung (pre-coke) ist es möglich, die Aktivität des Katalysators für ein Selektivitätsoptimum des gewünschten Reaktionsproduktes einzustellen.

Bevorzugt ist die Verwendung einer Mineralsäure, besonders bevorzugt von Schwefelsäure als Katalysator.

Der Katalysator wird dabei in Mengen von 0,02 bis 0,03 Mol, bevorzugt in Mengen von 0,021 bis 0,025 Mol, z.B. 0,023 Mol, pro Mol 2-Keto-L-gulonsäure eingesetzt.

In einer weiteren Ausführungsform beträgt die Reaktionstemperatur der gesamten Veresterung, d.h. während der Vorveresterung und der Veresterung in der Reaktivkolonne, 50°C bis 120°C, bevorzugt 80°C bis 90°C.

Vorzugsweise wird die Vorveresterung in einem kontinuierlichen Verfahren durchgeführt, z.B. in einem kontinuierlich durchströmten Rührbehälter oder anderen Reaktor.

Die Verweilzeit in dem Reaktor hängt u.a. von der Temperatur und dem zu erreichenden Veresterungsgrad ab. In einer bevorzugten Ausführungsform wird eine Verweilzeit von 1 bis 3 Stunden, bevorzugt von 1,5 h, gewählt bei einer Reaktionstemperatur in der Vorveresterung von 65°C bis 120°C, bevorzugt 80°C bis 90°C.

Vorzugsweise wird die Vorveresterung in dem erfindungsgemäßen Verfahren bei 65°C bis 120°C durchgeführt, besonders bevorzugt sind 80°C bis 90°C. Die Wahl der Temperatur hängt von der gewünschten Reaktionsdauer und dem gewünschten zu erzielenden Veresterungsgrad ab und liegt im Wissen eines durchschnittlichen Fachmanns.

Folglich betrifft die Erfindung bevorzugt ein Verfahren, das unter folgenden Bedingungen durchgeführt wird:
mittlere Verweilzeit der wässrigen KGS in dem Rührbehälter 1 bis 3 h, bevorzugt 1 bis 1,5 h, bei einer Reaktionstemperatur von 65°C bis 120°C, bevorzugt 75°C bis 95°C, mehr bevorzugt 80°C bis 90°C; bei einem Masseverhältnis von KGS zu C₄-C₁₀-Alkohol 1:1 bis 5:1, bevorzugt 2,5:1 bis 3,5:1; und bei Reaktionstemperaturen während des gesamten Verfahrens von 50°C bis 120°C, bevorzugt 80°C bis 90°C, wobei ein Veresterungsgrad von 20 bis 70 % erreicht wird. Bevorzugt werden die Bedingungen so gewählt, dass der Veresterungsgrad 30 % bis 50 % beträgt.

Nach dem erfindungsgemäßen Vorveresterungsschritt wird das Produkt erfindungsgemäß im zweiten Schritt einem kontinuierlichen Rektifikationsapparat zugeführt und dort weiter verestert. Der Rektifikationsapparat ist vorzugsweise eine Boden-, Füllkörper-oder Packungskolonne. Durch den hierin beschriebenen erfindungsgemäßen Vorveresterungsschritt wird vermieden, dass es zu einem Ausfall von KGS in dem Apparat an den Einbauten wie Böden, Füllkörper, oder Packungen zu Feststoffablagerungen kommt. Die dem Apparat zugeführte Lösung hat bereits einen hohen Anteil an KGS-Ester und somit eine höhere Löslichkeit für KGS.

In einer Ausführungsform wird folglich in dem erfindungsgemäßen Verfahren der kontinuierliche Rektifikationsapparat (2) mit einem Verdampfer (3) und einem Kondensator (4) ausgestattet, in einer besonders bevorzugten Ausführungsform zusätzlich mit einem Phasentrennapparat (5) und vorzugsweise einer Vakuumeinrichtung (6).

In einer weiteren Ausführungsform wird der erste Rektifikationsapparat durch einen zweiten Rektifikationsapparat am Voreresterungsapparat ergänzt. Folglich ist in einer Ausführungsform der Vorveresterungsreaktor (1) mit einer zusätzlichen Kolonne (7), einem zusätzlichen Verdampfer (8), gegebenenfalls einem zusätzlichen Kondensator (9) und gegebenenfalls einem zusätzlichen Phasentrennapparat (10) ausgestattet.

Rektifikationskolonnen sind im Stand der Technik umfangreich beschrieben, z.B. in Thermische Trennverfahren, K. Sattler, VCH Verlagsgesellschaft, Weinheim, 1995, insbesondere Kapitel 2 und Kapitel 7 oder in DE 199 38 980, auf deren Ausführungen ausdrücklich bezug genommen wird.

Bevorzugt werden Rektifikationskolonnen verwendet, die zur Durchführung der erfindungsgemäßen Veresterungsreaktion besonders geeignet sind. Diese Kolonnen zeichnen sich durch eine hohe Verweilzeit der Reaktionslösung in der jeweiligen Stufe aus. So können vorteilhaft z.B. Kolonnen verwendet werden, die einen hohen Flüssigkeits-"hold-up" haben, wie z.B. bei hochaufgestauten Böden einer Bodenkolonne. Folglich eignen sich für das erfindungsgemäße Verfahren insbesondere auch Kolonnen, die nur wenige Trennstufen aufweisen, solange eine geeignete Verweildaher sicher gestellt ist. Besonders vorteilhaft wird insbesondere eine geringe Temperatur eingesetzt, z.B. unter 100°C, vorzugsweise 80°C bis 90°C.

Die erfindungsgemäße Ausführungsform führt zu einem Produkt mit geringen Nebenprodukten und guten Farbzahlen.

In einer Ausführungsform wird die Vorveresterung ohne Wasserentfernung durchgeführt, jedoch kann in der Vorveresterung auch durch den Einsatz einer zweiten Rektifikationskolonne während der Reaktion Wasser abdestilliert werden, wenn sich die ausgefällte KGS in der Reaktionslösung während der Veresterung wieder gelöst hat.

Vorteilhafterweise kann in dem erfindungsgemäßen Verfahren die wässrige KGS-Lösung vor Eintritt in den Vorveresterungsreaktor bis an die Löslichkeitsgrenze von KGS oder darüber hinaus aufkonzentriert wird. Die Löslichkeitsgrenze von KGS hängt von der Temperatur der Lösung und vom erhöhten Estergehalt ab. Vorzugsweise wird die KGS-Lösung auf einen KGS-Massenanteil bis an die Löslichkeitsgrenze bei 30°C bis 70°C, mehr bevorzugt bei 40°C bis 60°C, am meisten bevorzugt bei 45°C bis 55°C aufkonzentriert. Bei einer Temperatur von 50°C ist z.B. die Löslichkeitsgrenze bei 50 % KGS-Massenanteil erreicht.

Wird die wässrige KGS-Lösung vor der Veresterung über die Löslichkeitsgrenze hinaus aufkonzentriert, entsteht eine Suspension. Diese Suspension kann dann erfindungsgemäß verestert werden, wobei geeignete Reaktionsapparate nach dem Wissen des Fachmanns einzusetzen sind, z.B. einen Vorveresterungsreaktor, wie in den Beispielen beschrieben. Durch die fortschreitende Vorveresterung und den erhöhten Ester-Anteil in der Lösung löst sich das ausgefällte KGS wieder. Es kommt somit auch dann nicht zu einer Feststoffablagerung in der folgenden Rektifikationskolonne. Vielmehr kann das erfindungsgemäße Verfahren so besonders wirtschaftlich betrieben werden, da der Eintrag in die Veresterungsreaktion eine höhere KGS-Konzentration aufweisen kann als dies nach im Stand der Technik beschriebenen Verfahren möglich ist. Dies führt zu Einsparungen insbesondere an Energie. Der Abzug in die Destillationskolonne erfolgt so, dass kein Feststoff übertragen wird, z.B. aus dem oberen Bereich des Vorveresterungsreaktors knapp unter der Flüssigkeitsoberfläche.

In einer weiteren Ausführungsform ist das vorliegende erfindungsgemäße Verfahren Teil eines Verfahrens zur Herstellung von Ascorbinsäure, wobei der hergestellte 2-Keto-L-gulonsäure-C₄-C₁₀-alkylester in einem oder mehr Schritten zu L-Ascorbinsäure umgesetzt wird, z.B. mittels basischen oder sauer katalysierten Lactonisierung.

Besonders bevorzugt umfasst das erfindungsgemäße Verfahren folglich die folgenden Schritte:
a) Herstellen einer wässrigen KGS-Lösung, z.B. aus einer Fementationsbrüche;
b) Aufkonzentrieren der wässrigen KGS-Lösung bei 30°C bis 60°C, vorteilhaft bei 50°C;
c) Vorveresterung der KGS-Lösung mit einem C₄-C₁₀-Alkylalkohol, bevorzugt Butanol nach dem oben beschriebenen Verfahren;
d) Entfernen des Wassers mit einem C₄- bis C₁₀-Alkyl-Alkohol in einem Rektifikationsapparat nach dem oben beschriebenen Verfahren;
e) Aufkonzentrieren des hergestellten KGS-Esters; ggf. Umestern des KGS-C₄-C₁₀-Alkylalkoholesters mit einem C₁-C₃-Alkylalkohol, insbesondere Methanol;
f) Lactonisieren des Esters zu L-Ascorbinsäure; und
g) Isolieren der freien L-Ascorbinsäure;
wobei jeder dieser Schritte außer (c) und (d) optional ist oder von weiteren Zwischenstufen begleitet werden kann, wie sie im Stand der Technik beschrieben sind, z.B. Einengen und Aufkonzentrieren der Lösungen, Filtrieren, wie z.B. Mikro- oder Ultrafiltration, Fällen, Kristallisieren, Extrahieren, Freisetzen von Säuren aus Salzen. Bevorzugt enthält das erfindungsgemäße Verfahren in einer Ausführungsform alle genannten Schritte (a) bis (g).

Eine geeignete KGS-Lösung kann z.B. durch die Aufarbeitung einer Fermentationsbrühe von KGS-produzierenden Mikroorganismen erfolgen.

Die Aufarbeitung einer Fermentationslösung kann nach den im Stand der Technik beschriebenen Verfahren erfolgen, z.B. durch Filtration oder durch Isolierung von Na-KGS durch Kristallisation oder Fällung.

Eine Einengung oder Aufkonzentrierung einer Lösung, insbesondere der KGS-Lösung, kann nach im Stand der Technik bekannten Verfahren erfolgen, beispielsweise durch Verdampfung oder durch Osmose, z.B. durch reverse Osmose. Vorzugsweise wird unter milden Bedingungen gearbeitet, insbesondere bei Temperaturen von 40°C bis 60°C und bei verringertem Druck.

Die Lactonisierung kann sauer oder basisch katalysiert erfolgen. Entsprechende Verfahren sind im Stand der Technik umfangreich beschrieben.

Auf die hierin aufgeführten Druckschriften wird ausdrücklich bezug genommen.

Die Erfindung wird anhand der folgenden Abbildungen verdeutlich:

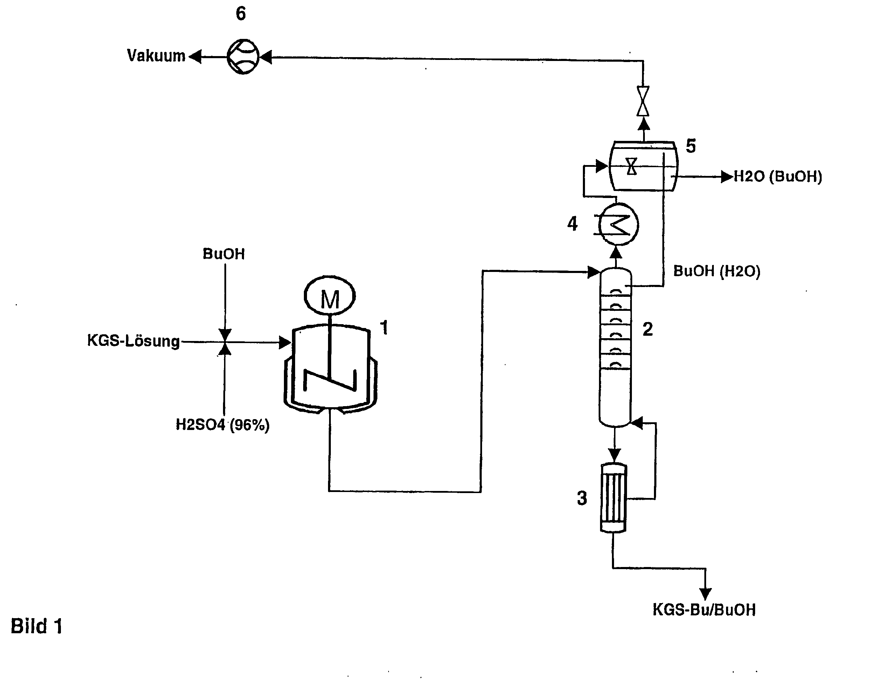

Abbildung 1 zeigt das erfindungsgemäße Verfahren ohne Wasser-Abzug bei der Vorveresterung mit einem Rektifikationsapparat: Vorveresterungsreaktor (1) mit folgendem Rektifikationsapparat (2) mit einem Verdampfer (3), einem Kondensator (4), einem Phasentrennapparat (5) und einer Vakuumeinrichtung (6).

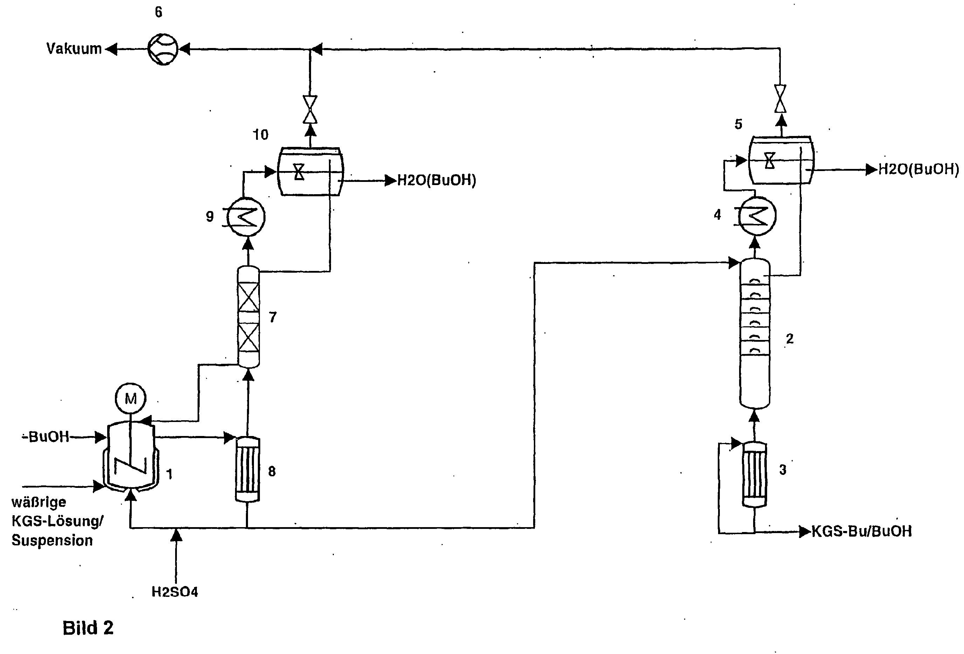

Abbildung 2 zeigt das erfindungsgemäße Verfahren mit Wasser-Abzug bei der Vorveresterung mit einem zweiten Rektifikationsapparat: Vorveresterungsreaktor (1) mit folgendem Rektifikationsapparat (3) mit einem Verdampfer (2), einem Kondensator (4), einem Phasentrennapparat (5) und einer Vakuumeinrichtung (6), wobei der Vorveresterungsreaktor (1) mit einem zusätzlichen Rektifikationsapparat (7), einem zusätzlichen Verdampfer (8), einem zusätzlichen Kondensator (9) und einem zusätzlichen Phasentrennapparat (10) ausgestattet ist.

Die vorliegende Erfindung wird durch die folgenden Beispiele verdeutlicht, ohne dass diese als einschränkend gelten sollen.

### Beispiel 1

Eine allgemeine Ausführungsform des erfindungsgemäßen Verfahren ist in Abbildung 1 dargestellt: Eine bei 50°C bis an die Löslichkeitsgrenze aufkonzentrierte wässrige KGS-Lösung (50 % KGS-Massenanteil) wird mit n-Butanol, im Massenverhältnis 1:1 bis 3:1, bevorzugt 1,5:1 bis 2:1 und mit katalytischen Mengen an konzentrierter Schwefelsäure gemischt und in einen über die Wandung beheizten, kontinuierlich durchströmten Rührbehälter oder anderen Reaktor (1), dessen Volumen so ausgelegt ist, dass die Verweilzeit 1 bis 3, bevorzugt 1,5 h, beträgt, zugeführt. Die Heizung des Reaktors 1 ist so eingestellt, dass sich im Inneren des Reaktors 65 bis 120°C, bevorzugt 80 bis 90°C, einstellen. Im Reaktor 1 wird so ein Veresterungsgrad von 30 bis 60 % erreicht. Das aus 1 austretende Gemisch wird einem mehrstufigen Rektifikationsapparat (2), z.B. einer Boden- oder Packungskolonne zugeführt. Der kontinuierliche Rektifikationsapparat (2) ist mit einem Verdampfer (3), einem Kondensator (4) und einem Phasentrennapparat (5), sowie einer Vakuumeinrichtung (6) ausgestattet. Das eintretende Gemisch läuft im Apparat (1) unter Schwerkrafteinfluss nach unten, während die überwiegend n-BuOH enthaltenden Dämpfe aus dem Verdampfer (3) der Flüssigkeit entgegenströmen. Die Konzentration an Wasser in der Flüssigkeit nimmt auf dem Weg nach unten, von Trennstufe zu Trennstufe ab, wodurch die Veresterungsreaktion fortschreitet (Reaktivdestillation). Am Kopf des Apparates 2 werden die azeotrop zusammengesetzten Brüden kondensiert und in einem Flüssig-Flüssig-Phasentrennapparat (5) mechanisch in eine überwiegend BuOH-haltige und eine überwiegend wasserhaltige Phase getrennt. Die wasserhaltige Phase wird abgezogen und einer Abwasserbehandlung, ggf. mit BuOH-Rückgewinnung, zugeführt, die BuOH-Phase als Rücklauf wieder auf den Rektifikationsapparat 1 zurückgegeben. Die Temperatur im Verdampfer 3 wird durch Anlegen eines geeigneten Vakuums (6) auf 65 bis 110°C, bevorzugt 80 bis 90°C eingestellt. Am Sumpf des Verdampfers 3 wird ein nahezu wasserfreies Gemisch aus KGS-butylester und BuOH abgezogen. Durch den erfindungsgemäßen Vorreaktor kommt es üblicherweise zu keinerlei Feststoffausfall im Rektifikationsapparat 2.

### Beispiel 2

Abbildung 2 zeigt eine weitere Ausführungsform des erfindungsgemäßen Verfahrens: Hier wird der Vorveresterungsreaktor 1 mit einer zusätzlichen Kolonne 2, einem zusätzlichen Verdampfer 3, einem zusätzlichen Kondensator 4 und einem zusätzlichen Phasentrennapparat 5 ausgestattet und so bereits in der Vorveresterung eine teilweise Wasserentfernung und damit ein höherer Veresterungsgrad erreicht. Da die Vorveresterung weiterhin im Rührbehälter erfolgt (KGS ist ein Schwersieder), können die Einbauten der Kolonne 2 nicht mit Feststoff belegt werden.

### Beispiel 3

Eine wässrige KGS-Lösungen wurde durch mehrstufige Wasserabdampfungen (z.B. in Fallfilmverdampfern), in der letzten Stufe bei ca. 50°C und entsprechendem Vakuum bis zu einer Konzentration von 50 % KGS (m/m) konzentriert, so dass kein Feststoff ausfällt. Diese Lösung wurde z.B. in einem beheizten Rührbehälter im Masseverhältnis 1,73:1 mit n-Butanol (n-BuOH) gemischt, dann eine geringe Menge Schwefelsäure (H₂SO₄) als Katalysator hinzugegeben (ca. 1.% Massenanteil bez. auf KGS). Die klare, feststofffreie Mischung wurde bei 85°C 1 bis 1,5 h lang verestert. Trotz des relativ hohen Wassermassenanteiles in der Mischung von ca. 18 % wurde ein KGS-Umsatz zum KGS-butylester von 45 bis 50 % erreicht. Bei der weiteren Veresterung des Gemisches in einer Reaktivdestillation (z.B. gemäß DE 199 38 980) tritt kein Feststoffausfall auf.

### Beispiel 4

In einem von außen durch ein Ölbad beheizten Rührbehälter (max. Füllvolumen 100 cm³) wurden 23 g trockene, calzinierte KGS in 23 g Wasser bei 85°C vollständig innerhalb weniger Minuten gelöst. Dann wurden 80 g auf 85°C vorgewärmtes n-Butanol, sowie 0,3 g konzentrierte Schwefelsäure (96 %) zugeben und die Mischung 1,5 h unter Rückfluß gerührt. Die KGS löste sich vollständig. Anschließend wurde der Massenanteil an KGS-Butylester im Gemisch durch HPLC bestimmt, es wurde ein Massenanteil von 10,68 % gemessen. Dies entspricht einem Esterbildungsgrad von 45,5 %.

### Beispiel 5

Die Apparatur wurde nun um einen wassergekühlten Kondensator und eine Vakuumpumpe erweitert. In einem zweiten, bis zum oben beschriebenen Punkt identisch durchgeführten Experiment, wurde, ohne Probenentnahme, durch Anlegen eines Vakuums, bei 85°C, dicht am Siedepunkt, innerhalb von 30 min 75 g der Flüssigkeit abdestilliert. Die Flüssigkeitsmischung im Rührbehälter blieb bis zum Ende klar, es war kein Feststoffausfall zu beobachten. Eine Bestimmung des Restwassergehaltes nach Karl Fischer ergab einen Restwassergehalt von 0,4 % (m/m).

### Beispiel 6

In einem weiteren Experiment wurde wie im zweiten Beispiel 23 g trockene, calzinierte KGS in 23 g Wasser bei 85°C vollständig innerhalb weniger Minuten gelöst. Dann wurden 80 g auf 85°C vorgewärmtes n-Butanol, sowie 0,3 g konzentrierte Schwefelsäure (96 %) zugeben und sofort, durch Anlegen des Vakuums, mit der Destillation begonnen. Es kam zwischenzeitlich zur Eintrübung durch Feststoffausfall. Der Feststoff löste sich im weiteren Fortgang wieder auf.

## Patentansprüche

1. Verfahren zur Herstellung von 2-Keto-L-gulonsäure-C₄-C₁₀-alkylester durch Veresterung von 2-Keto-L-gulonsäure (KGS) mit einem gesättigten, verzweigten oder unverzweigten C₄-C₁₀-Alkohol, **dadurch gekennzeichnet, dass** in einer Vorveresterung eine wässrige KGS-Lösung mit einem C₄-C₁₀-Alkohol unter saurer Katalyse bis zu einem Veresterungsgrad von 20 % bis 70 % umgesetzt wird und das Produkt in einem kontinuierlichen Rektifikationsapparat mit einem C₄-C₁₀-Alkohol entwässert wird, wodurch die Veresterungsreaktion fortschreitet.

2. verfahren nach Anspruch 1, wobei der Alkohol ein gesättigter, verzweigter oder unverzweigter Alkylalkohol mit 4 bis 10 Kohlenstoffatomen, vorzugsweise n-Butanol, ist.

3. Verfahren nach Anspruch 1 oder 2, wobei in der Vorveresterung der Alkohol in einem Massenverhältnis zum KGS-Anteil in der wässrigen Lösung von 1:1 bis 5:1 eingesetzt wird.

4. Verfahren nach einem der Ansprüche 1 bis 3, wobei der Katalysator ein saurer heterogener oder homogener Katalysator ist.

5. Verfahren nach einem der Ansprüche 1 bis 4, wobei der Katalysator eine Mineralsäure ist.

6. Verfahren nach einem der Ansprüche 1 bis 5, wobei die Vorveresterung in einem kontinuierlich durchströmten Rührbehälter durchgeführt wird.

7. Verfahren nach einem der Ansprüche 1 bis 5, wobei das Verfahren unter folgenden Bedingungen durchgeführt wird:
a) mittlere Verweilzeit der wässrigen KGS in der Vorveresterung 1 bis 3 h,
b) Reaktionstemperatur in der Vorveresterung von 65°C bis 120°C; und/oder
c) Masseverhältnis von KGS-Anteil zu C₄-C₁₀-Alkohol 1:1 bis 5:1; und/oder
d) Reaktionstemperaturen während des gesamten Verfahrens von 50°C bis 120°C und/oder
e) Verwendung von 0,02 bis 0,03 mol Schwefelsäure per mol KGS als Katalysator.

8. Verfahren nach einem der Ansprüche 1 bis 7, wobei die wässrige KGS-Lösung vor Eintritt in den Vorveresterungsreaktor bis an die Löslichkeitsgrenze von KGS aufkonzentriert wird.

9. Verfahren nach einem der Ansprüche 1 bis 7, wobei die wässrige KGS-Lösung vor Eintritt in den Vorveresterungsreaktor über die Löslichkeitsgrenze von KGS hinaus aufkonzentriert wird.

10. Verfahren nach einem der Ansprüche 1 bis 9, wobei der kontinuierliche Rektifikationsapparat (2) mit einem Verdampfer (3) und einem Kondensator (4), sowie vorzugsweise mit einem Phasentrennapparat (5) und/oder einer Vakuumeinrichtung (6) ausgestattet ist.

11. Verfahren nach einem der Ansprüche 1 bis 9, wobei der Vorveresterungsreaktor (1) mit einer zusätzlichen Kolonne (7), einem zusätzlichen Verdampfer (8)und einem zusätzlichen Kondensator (9), sowie vorzugsweise mit einem zusätzlichen Phasentrennapparat (10), ausgestattet ist.

12. Verfahren zur Herstellung von Ascorbinsäure, wobei das Verfahren das Verfahren nach einem der Ansprüche 1 bis 11 umfasst und der hergestellte 2-Keto-L-gulonsäure-C₄-C₁₀-alkylester in einem oder mehr Schritten zu L-Ascorbinsäure umgesetzt wird.

## Claims

1. A process for preparing 2-keto-L-gulonic C₄-C₁₀-alkyl esters by esterifying 2-keto-L-gulonic acid (KGA) with a saturated, branched or unbranched C₄-C₁₀-alcohol, which comprises, in a preliminary esterification, reacting an aqueous KGA solution with a C₄-C₁₀-alcohol under acid catalysis up to a degree of esterification of from 20% to 70% and dehydrating the product in a continuous rectification apparatus using a C₄-C₁₀-alcohol, as a result of which the esterification reaction advances.

2. The process according to claim 1, wherein the alcohol is a saturated, branched or unbranched alkyl alcohol having from 4 to 10 carbons, preferably n-butanol.

3. The process according to claim 1 or 2, wherein, in the preliminary esterification, the alcohol is used in a mass ratio to the KGA content in the aqueous solution of from 1:1 to 5:1.

4. The process according to any of claims 1 to 3, wherein the catalyst is an acid heterogeneous or homogeneous catalyst.

5. The process according to any of claims 1 to 4, wherein the catalyst is a mineral acid.

6. The process according to any of claims 1 to 5, wherein the preliminary esterification is carried out in a continuous-flow stirred tank.

7. The process according to any of claims 1 to 5, which is carried out under the following conditions:
a) mean residence time of the aqueous KGA in the preliminary esterification from 1 to 3 h,
b) reaction temperature in the preliminary esterification from 65°C to 120°C; and/or
c) mass ratio of KGA content to C₄-C₁₀-alcohol from 1:1 to 5:1; and/or
d) reaction temperatures during the entire process from 50°C to 120°C and/or
e) use of from 0.02 to 0.03 mol of sulfuric acid per mole of KGA as catalyst.

8. The process according to any of claims 1 to 7, wherein the aqueous KGA solution, before entry into the preliminary esterification reactor, is concentrated up to the solubility limit of KGA.

9. The process according to any of claims 1 to 7, wherein the aqueous KGA solution, before entry into the preliminary esterification reactor, is concentrated to above the solubility limit of KGA.

10. The process according to any of claims 1 to 9, wherein the continuous rectification apparatus (2) is equipped with an evaporator (3) and a condenser (4), and also preferably with a phase-separation apparatus (5) and/or a vacuum system (6).

11. The process according to any of claims 1 to 9, wherein the preliminary esterification reactor (1) is equipped with an additional column (7), an additional evaporator (8) and an additional condenser (9) and also preferably with an additional phase-separation apparatus (10).

12. A process for preparing ascorbic acid, which comprises the process according to any of claims 1 to 11 and the 2-keto-L-gulonic C₄-C₁₀-alkyl ester prepared being converted to L-ascorbic acid in one or more steps.

## Revendications

1. Procédé de préparation d'ester alkylique en C₄-C₁₀ d'acide 2-céto-L-gulonique par estérification d'acide 2-céto-L-gulonique (CGA) avec un alcool en C₄-C₁₀ saturé, ramifié ou non ramifié, **caractérisé en ce que**, dans une estérification préalable, on fait réagir une solution aqueuse de CGA avec un alcool en C₄-C₁₀, sous catalyse acide, jusqu'à un degré d'estérification de 20 % à 70 % et on déshydrate le produit avec un alcool en C₄-C₁₀ dans un appareil de rectification continue, ce qui permet une poursuite de la réaction d'estérification.

2. Procédé suivant la revendication 1, dans lequel l'alcool est un alcool alkylique saturé, ramifié ou non ramifié, comportant 4 à 10 atomes de carbone, de préférence du n-butanol.

3. Procédé suivant la revendication 1 ou 2, dans lequel, dans l'estérification préalable, on met en oeuvre un rapport pondéral entre l'alcool et la fraction de CGA dans la solution aqueuse de 1/1 à 5/1.

4. Procédé suivant l'un des revendications 1 à 3, **caractérisé en ce que** le catalyseur est un catalyseur homogène ou hétérogène acide.

5. Procédé suivant l'une des revendications 1 à 4, dans lequel le catalyseur est un acide minéral.

6. Procédé suivant l'une des revendications 1 à 5, dans lequel l'estérification préalable est effectuée dans un récipient à agitation traversé par un écoulement continu.

7. Procédé suivant l'une des revendications 1 à 5, dans lequel le procédé est effectué dans les conditions suivantes :
a) durée de séjour moyenne du CGA aqueux dans l'estérification préalable de 1 à 3 heures,
b) température réactionnelle de l'estérification préalable de 65°C à 120°C, et/ou
c) rapport pondéral entre la fraction de CGA et l'alcool en C₄-C₁₀ de 1/1 à 5/1, et/ou
d) températures réactionnelles pendant la totalité du procédé de 50°C à 120°C, et/ou
e) utilisation de 0,02 à 0,03 mole d'acide sulfurique par mole de CGA, à titre de catalyseur.

8. Procédé suivant l'une des revendications 1 à 7, dans lequel la solution aqueuse de CGA est, avant l'entrée dans le réacteur d'estérification préalable, concentrée jusqu'à la limite de solubilité du CGA.

9. Procédé suivant l'une des revendications 1 à 7, dans lequel la solution aqueuse de CGA est, avant l'entrée dans le réacteur d'estérification préalable, concentrée au-delà de la limite de solubilité du CGA.

10. Procédé suivant l'une des revendications 1 à 9, dans lequel l'appareil de rectification continue (2) est équipé d'un évaporateur (3) et d'un condenseur (4), ainsi que de préférence d'un appareil de séparation de phases (5) et/ou d'un dispositif à vide (6).

11. Procédé suivant l'une des revendications 1 à 9, dans lequel le réacteur d'estérification préalable (1) est équipé d'une colonne additionnelle (7), d'un évaporateur additionnel (8) et d'un condenseur additionnel (9) ainsi que, de préférence, d'un appareil de séparation de phases additionnel (10).

12. Procédé de préparation d'acide ascorbique, dans lequel le procédé comporte le procédé suivant l'une des revendications 1 à 11, et l'ester alkylique en C₄-C₁₀ d'acide 2-céto-L-gulonique préparé est mis à réagir en une ou plusieurs étapes pour former de l'acide L-ascorbique.
